(19) Europäisches Patentamt / European Patent Office / Office européen des brevets

(11) **EP 1 412 083 B1**

(12) **EUROPÄISCHE PATENTSCHRIFT**

(45) Veröffentlichungstag und Bekanntmachung des Hinweises auf die Patenterteilung:
**15.10.2008 Patentblatt 2008/42**

(21) Anmeldenummer: **02747366.9**

(22) Anmeldetag: **10.06.2002**

(51) Int Cl.:
*B01J 23/46* *(2006.01)*   *B01J 37/18* *(2006.01)*
*C07C 31/26* *(2006.01)*   *C07C 29/141* *(2006.01)*

(86) Internationale Anmeldenummer:
**PCT/EP2002/006349**

(87) Internationale Veröffentlichungsnummer:
**WO 2002/100539 (19.12.2002 Gazette 2002/51)**

(54) **VERFAHREN ZUR HERSTELLUNG VON SORBIT DURCH HYDRIERUNG GEEIGNETER MONOSACCHARIDE AN EINEM RU/SIO2-KATALYSATOR**

METHOD FOR THE PRODUCTION OF SORBIT BY HYDROGENATION OF SUITABLE MONOSACCHARIDES ON A RU/SIO2 CATALYST

PROCEDE DE FABRICATION DE SORBITOL PAR HYDROGENATION DE MONOSACCHARIDES SUR UN CATALYSEUR RU/SIO2

(84) Benannte Vertragsstaaten:
**AT BE CH CY DE DK ES FI FR GB GR IE IT LI LU MC NL PT SE TR**

(30) Priorität: **11.06.2001 DE 10128203**

(43) Veröffentlichungstag der Anmeldung:
**28.04.2004 Patentblatt 2004/18**

(73) Patentinhaber: **BASF SE**
**67056 Ludwigshafen (DE)**

(72) Erfinder:
• VANOPPEN, Dominic
  B-2950 Kapellen (BE)
• MAAS-BRUNNER, Melanie
  68165 Mannheim (DE)
• KAMMEL, Ulrich
  67346 Speyer (DE)
• ARNDT, Jan-Dirk
  68167 Mannheim (DE)

(74) Vertreter: **Reitstötter - Kinzebach**
**Ludwigsplatz 4**
**67059 Ludwigshafen (DE)**

(56) Entgegenhaltungen:
EP-A- 0 992 475       FR-A- 2 526 782
US-A- 4 072 628       US-A- 4 950 812
US-A- 5 334 790

• HALLER GARY L. ET AL: "The Effect of Silica Support Texture and Anion of Impregnating Solution on Ru Dispersion on Ru-Cu Interaction " JOURNAL OF CATALYSIS, Bd. 84, 1983, Seiten 477-479, XP009005889

**Beschreibung**

Die vorliegende Erfindung betrifft ein Verfahren zur Herstellung von Sorbit durch katalytische Hydrierung eines geeigneten Mono saccharids.

Die großtechnische Herstellung von Sorbit erfolgt durch katalytische Hydrierung von Glukose, Fruktose, Saccharose oder Invertzukker (siehe H. Schiweck et al. "Sugar Alcohols" in Ullmann's Encyclopedia of Industrial Chemistry, 5th ed. on CD-ROM). Zu diesem Zweck wurden als Katalysatoren bislang in erster Linie Nickel-Katalysatoren, wie z.B. Nickel-Trägerkatalysatoren oder Raney-Nickel eingesetzt. Verschiedentlich wurde auch über den Einsatz von Ruthenium-haltigen Katalysatoren für diesen Zweck berichtet. In der Regel handelt es sich bei Ruthenium-Katalysatoren um sogenannte Trägerkatalysatoren, die Ruthenium auf einem oxidischen oder organischen Träger wie Kohle enthalten.

So beschreiben die US 4,380,680, US 4,487,980, US 4,413,152 und die US 4,471,144 die Herstellung von Sorbit durch katalytische Hydrierung von Glucose, in denen Katalysatoren eingesetzt werden, die Ruthenium auf einem unter hydrothermalen Bedingungen stabilen Trägermaterial enthalten. Als hydrothermale Trägermaterialien werden alpha-Aluminiumoxid (US 4,380,680), Titan(IV)oxid (US 4,487,980), mit Titan(IV)halogenid behandeltes Aluminiumoxid (US 4,413,152) und theta-Aluminiumoxid (US 4,471,144) vorgeschlagen.

Aus der US 4,503,274 sind Katalysatoren für die Hydrierung von Glucose zu Sorbit bekannt, die durch Imprägnieren eines unter hydrothermalen Bedingungen stabilen Trägers mit einer wässrigen Rutheniumhalogenid-Lösung und anschließendes Hydrieren des Feststoffs bei Temperaturen im Bereich von 100 bis 300°C hergestellt werden.

Die US 3,963,788 beschreibt die Hydrierung von Mais-Stärke-Hydrolysaten zu Sorbit an Ruthenium-Katalysatoren, in denen das Ruthenium mit einem speziellen Zeolithen auf Basis eines Alumosilikats geträgert wurde. Die US 3,963,789 schlägt als Träger für Ruthenium-Katalysatoren kristalline Alumosilikat-Tone, insbesondere Montmorillonit vor.

Die FR-A 2526782 beschreibt die Verwendung eines durch Umsetzung von Natriumchlorid und Ruthenium via $Na_2RuCl_6$ hergestellten Rutheniumchlorids zur Herstellung von auf Siliziumdioxid geträgerten Ruthenium-Katalysatoren für die Hydrierung von Mono- und Oligosacchariden, z.B. für die Herstellung von Sorbit.

Die parallele Anmeldung WO 02/100537 betrifft Rutheniumkatalysatoren, die erhältlich sind durch: i) ein oder mehrfaches Behandeln eines Trägermaterials auf Basis von amorphem siliziumdioxid mit einer halogenfreien wässrigen Lösung einer niedermolekularen Rutheniumverbindung und anschließendes Trockenen des behandelten Trägermaterials bei einer Temperatur unterhalb 200°C, ii) Reduktion des in i) erhaltenen Feststoffs mit Wasserstoff bei einer Temperatur im Bereich von 100 bis 350°C, wobei man Schritt ii) unmittelbar in Anschluss an Schritt i) durchführt. Die Katalysatoren werden zur katalytischen Hydrierung von Mono- oder Disacchariden eingesetzt.

Die parallele Anmeldung WO 02/100536 betrifft ein Verfahren zur Hydrierung ein oder mehrkerniger Aromaten unter Verwendung derartiger Katalysatoren.

Die parallele Anmeldung WO 02/100538 betrifft die Verwendung derartiger Katalysatoren zur Herstellung cycloaliphatischer Verbindungen, die Seitenketten mit Epoxidgruppen aufweisen, durch katalytische Hydrierung entsprechender aromatischer Verbindungen.

Die aus dem Stand der Technik bekannten Verfahren für die Herstellung von Sorbit durch Hydrierung an Ruthenium-Katalysatoren liefern aufgrund der nur mäßigen Aktivität der Katalysatoren Sobit nur mit mäßigen Raum-Zeit-Ausbeuten, bezogen auf den eingesetzten Katalysator. Angesichts der hohen Kosten für Ruthenium lässt daher die Wirtschaftlichkeit dieser Verfahren zu wünschen übrig. Zudem sind die Selektivitäten der Katalysatoren nicht ausreichend, so dass zusätzlicher Aufwand beim Isolieren der Wertprodukte erforderlich ist. Insbesondere wird häufig eine Epimerisierung der Hydroxygruppen beobachtet.

Der vorliegenden Erfindung liegt daher die Aufgabe zugrunde, ein Verfahren zur Herstellung von Sorbit durch katalytische Hydrierung der entsprechenden Monosaccharide, die beim Hydrieren Sorbit bilden, bereitzustellen, das Sorbit mit bessern Raum-Zeit-Ausbeuten liefert und das eine vergleichbare oder bessere Selektivität hinsichtlich der Bildung vor Sorbit aufweist, d. h. bei dem nicht mehr oder vorzugsweise weniger Nebenprodukte anfallen als bei den Verfahren des Standes der Technik.

Diese Aufgabe wurde überraschenderweise gelöst durch Verwendung von Ruthenium-Katalysatoren, enthaltend Ruthenium auf einem Trägermaterial auf Basis von amorphem Siliziumdioxid, wobei der Gehalt an Ruthenium 0,2 bis 10 Gew.-%, bezogen auf das Trägermaterial, beträgt und das Trägermaterial zu wenigstens 90 Gew.-%, bezogen auf das Trägermaterial, aus Siliziumdioxid besteht und weniger als 10 Gew.-%, kristalline Siliziumdioxid-Phasen aufweist, wobei der Katalysator erhältlich ist durch:

i) ein oder mehrfaches Behandeln des Trägermaterials auf Basis von amorphem Siliziumdioxid mit einer wässrigen Lösung einer Rutheniumverbindung, ausgewählt unter Ruthenium((III)nitrosylnitrat, Ruthenium((III)acetat und Alkalimetallruthenaten(IV), wobei die wässrige Lösung, bezogen auf ihr Gesamtgewicht, weniger als 500 ppm Halogen enthält, und anschliessendes Trocknen des behandelten Trägermaterials bei einer Temperatur unterhalb 200°C,

ii) Reduktion des in i) erhaltenen Feststoffs mit Wasserstoff bei einer Temperatur im Bereich von 100 bis 350°C,

wobei man und Schritt ii) unmittelbar im Anschluss an Schritt i) durchführt.

Demnach betrifft die vorliegende Erfindung ein Verfahren zur Herstellung von Sorbit durch katalytische Hydrierung von einer wässrigen Lösung eines Monosaccharids das bei der Hydrierung Sorbit bildet, in flüssiger Phase, das dadurch gekennzeichnet ist, dass der Katalysator unter den vorstehend definierten Rutheniumkatalysatoren ausgewählt ist. Diese Katalysatoren sind neu und Gegenstand einer parallelen deutschen Patentanmeldung 10128205.2, sowie deren Nachanmeldung WO 02/100537.

Geeignete Ausgangsmaterialien für die Herstellung von Sorbit auf dem Wege der katalytischen Hydrierung sind Glucose, Fructose und Gulose, sowie Glucose enthaltende Produkte wie Invertzucker, der durch Hydrolyse von Saccharose erhalten wird. Bevorzugtes Ausgangsmaterial ist D-Glucose sowie Glucose-reiche Syrupe wie Maisstärke-, Weizenstärke- und Kartoffelstärke-Hydrolysate. Die Herstellung von D-Sorbit durch Hydrierung der D-Form der vorgenannten Monosaccharide ist von besonderem Interesse.

Es wird vermutet, dass die hohe Aktivität der im erfindungsgemäßen Verfahren eingesetzten Katalysatoren auf die besonderes gute Verteilung des Rutheniums auf der Oberfläche des Trägermaterials und auf die weitgehende Abwesenheit von Halogen im Trägermaterial zurückgeführt werden kann. Herstellungsbedingt liegt das Ruthenium in den erfindungsgemäßen Katalysatoren als metallisches Ruthenium vor. Elektronenmikroskopische Untersuchungen (TEM) der Katalysatoren haben gezeigt, dass das Ruthenium auf dem Trägermaterial in atomar-dipserser Form und/oder in Form von Ruthenium-Partikeln vorliegt, die nahezu ausschliesslich, d.h. zu mehr als 90 %, vorzugsweise zu mehr als 95 %, bezogen auf die Anzahl der sichtbaren Partikel, als isolierte Partikel mit Durchmessern unterhalb 10 nm, insbesondere unterhalb 7 nm vorliegen. Mit anderen Worten, der Katalysator enthält im Wesentlichen keine, d.h. zu weniger als 10 %, insbesondere weniger als 5 % Ruthenium-Partikel und/oder Agglomerate von Rutheniumpartikeln mit Durchmessern oberhalb 10 nm. Durch die Verwendung halogenfreier Rutheniumprekursoren und Lösungsmittel bei der Herstellung liegt der Chlorgehalt der erfindungsgemäß eingesetzten Katalysatoren zudem unterhalb 0,05 Gew.-% (< 500 ppm), bezogen auf das Gesamtgewicht des Katalysators. Hier und im Folgenden sind alle ppm-Angaben als Gewichtsanteile zu verstehen, soweit nichts anderes angegeben ist.

Ein wesentlicher Bestandteil der im erfindungsgemäßen Verfahren eingesetzten Katalysatoren ist das Trägermaterial auf Basis von amorphem Siliziumdioxid. Unter dem Begriff "amorph" versteht man in diesem Zusammenhang, dass der Anteil kristalliner Siliziumdioxid-Phasen weniger als 10 % des Trägermaterials ausmacht. Die zur Herstellung der Katalysatoren verwendeten Trägermaterialien können allerdings Überstrukturen aufweisen, die durch regelmässige Anordnung von Poren im Trägermaterial gebildet werden.

Als Trägermaterialien kommen grundsätzlich alle amorphen Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.-% des Trägermaterials auch ein anderes oxidisches Material sein können, z.B. MgO, CaO, $TiO_2$, $ZrO_2$, $Fe_2O_3$ oder Alkalimetalloxid. Es versteht sich von selbst, dass das eingesetzte Trägermaterial ebenfalls halogenfrei ist, d. h. der Halogengehalt beträgt weniger als 500 ppm bezogen auf das Gesamtgewicht des Trägermaterials. Vorzugsweise enthält das Trägermaterial nicht mehr als 1 Gew.-% und insbesondere nicht mehr als 0,5 Gew.-% und insbesondere keine nachweisbaren Mengen (< 500 ppm) an Aluminiumoxid, gerechnet als $Al_2O_3$. In einer bevorzugten Ausführungsform verwendet man Trägermaterialien, die weniger als 500 ppm $Fe_2O_3$ enthalten. Der Anteil an Alkalimetalloxid resultiert in der Regel aus der Herstellung des Trägermaterials und kann bis zu 2 Gew.-% betragen. Häufig beträgt er weniger als 1 Gew.-%. Geeignet sind auch Alkalimetalloxid-freie Träger (< 0,1 Gew.-%). Der Anteil an MgO, CaO, $TiO_2$ bzw. an $ZrO_2$ kann bis zu 10 Gew.-% des Trägermaterials ausmachen und beträgt vorzugsweise nicht mehr als 5 Gew.-%. Geeignet sind aber auch Trägermaterialien, die keine nachweisbaren Mengen dieser Metalloxide enthalten (< 0,1 Gew.-%).

Bevorzugt sind Trägermaterialien, die eine spezifische Oberfläche im Bereich von 50 bis 700 $m^2/g$, insbesondere im Bereich von 80 bis 600 $m^2/g$ und speziell im Bereich von 100 bis 600 $m^2/g$ aufweisen (BET-Oberfläche nach DIN 66131). Unter den pulverförmigen Trägermaterialien sind insbesondere solche bevorzugt, deren spezifische (BET) Oberfläche im Bereich von 200 bis 600 $m^2/g$ liegt. Bei Trägermaterial in Form von Formkörpern liegt die spezifische Oberfläche insbesondere im Bereich von 100 bis 300 $m^2/g$.

Geeignete amorphe Trägermaterialien auf Basis von Siliziumdioxid sind dem Fachmann geläufig und kommerziell erhältlich (siehe z.B. O.W. Flörke, "Silica" in Ullmann's Encyclopedia of Industrial Chemistry 5th ed. on CD-ROM). Sie können sowohl natürlichen Ursprungs als auch künstlich hergestellt worden sein. Beispiele für geeignete amorphe Trägermaterialien auf Basis von Siliziumdioxid sind Kieselgur, Kieselgele, pyrogene Kieselsäure und Fällungskieselsäure. In einer bevorzugten Ausführungsform der Erfindung weisen die Katalysatoren Kieselgele als Trägermaterialien auf.

Je nach Ausgestaltung des erfindungsgemäßen Verfahrens kann das Trägermaterial unterschiedliche Gestalt aufweisen. Sofern das Verfahren als Suspensionsverfahren ausgestaltet ist, wird man zur Herstellung der erfindungsgemäßen Katalysatoren üblicherweise das Trägermaterial in Form eines feinteiligen Pulvers einsetzen. Die Teilchengröße der Pulverteilchen liegt vorzugsweise im Bereich von 1 bis 200 $\mu$m und insbesondere im Bereich von 10 bis 100 $\mu$m. Bei Einsatz des Katalysators in Katalysatorfestbetten verwendet man üblicherweise Formkörper aus dem Trägermaterial, die z.B. durch Extrudieren, Strangpressen oder Tablettieren erhältlich sind und die z.B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 1 mm bis 25 mm. Häufig werden Katalysatorstränge mit Strangdurchmessern von 2 bis 5 mm und Stranglängen von 2 bis 25 mm eingesetzt.

Vorzugsweise liegt der Gehalt an Ruthenium in den Katalysatoren im Bereich von 0,2 bis 7 Gew.-% und insbesondere im Bereich von 0,4 bis 5 Gew.-%, jeweils bezogen auf das Gewicht des Trägermaterials und gerechnet als elementares Ruthenium.

Die Herstellung der im erfindungsgemäßen Verfahren eingesetzten Ruthenium-Katalysatoren erfolgt in der Regel dadurch, dass man zunächst das Trägermaterial mit einer halogenfreien wässrigen Lösung der Rutheniumverbindung, im Folgenden als (Ruthenium) prekursor bezeichnet, in einer Weise behandelt, dass die gewünschte Menge an Ruthenium vom Trägermaterial aufgenommen wird. Dieser Schritt wird im Folgenden auch als Tränken bezeichnet. Anschliessend wird der so behandelte Träger unter Einhaltung der oben angegebenen Temperaturobergrenzen getrocknet. Gegebenenfalls wird dann der so erhaltene Feststoff erneut mit der wässrigen Lösung des Rutheniumprekursors behandelt und erneut getrocknet. Dieser Vorgang wird so oft wiederholt, bis die vom Trägermaterial aufgenommene Menge an Rutheniumverbindung dem gewünschten Rutheniumgehalt im Katalysator entspricht.

Das Behandeln bzw. Tränken des Trägermaterials kann in unterschiedlicher weise erfolgen und richtet sich in bekannter Weise nach der Gestalt des Trägermaterials. Beispielsweise kann man das Trägermaterial mit der Prekursor-Lösung besprühen oder spülen oder das Trägermaterial in der Prekursor-Lösung suspendieren. Beispielsweise kann man das Trägermaterial in der wässrigen Lösung des Rutheniumprekursors suspendieren und nach einer gewissen zeit vom wässrigen Überstand abfiltrieren. Über die aufgenommene Flüssigkeitsmenge und die Ruthenium-Konzentration der Lösung kann dann der Rutheniumgehalt des Katalysators in einfacher Weise gesteuert werden. Das Tränken des Trägermaterials kann beispielsweise auch dadurch erfolgen, dass man den Träger mit einer definierten Menge der wässrigen Lösung des Rutheniumprekursors behandelt, die der maximalen Flüssigkeitsmenge entspricht, die das Trägermaterial aufnehmen kann. Zu diesem Zweck kann man beispielsweise das Trägermaterial mit der erforderlichen Flüssigkeitsmenge besprühen. Geeignete Apparaturen hierfür sind die zum Vermengen von Flüssigkeiten mit Feststoffen üblicherweise verwendeten Apparate (siehe Vauck/Müller, Grundoperationen chemischer Verfahrenstechnik, 10. Auflage, Deutscher Verlag für Grundstoffindustrie, 1994, S. 405 ff.) beispielsweise Taumeltrockner, Tränktrommeln, Trommelmischer, Schaufelmischer und dergleichen. Monolithische Träger werden üblicherweise mit den wässrigen Lösungen des Rutheniumprekursors gespült.

Die zum Tränken eingesetzten wässrigen Lösungen sind erfindungsgemäß halogenfrei, d.h. sie enthalten kein oder weniger als 500 ppm, vorzugsweise weniger als 100 ppm Halogen, bezogen auf das Gesamtgewicht der Lösung. Als Rutheniumprekursoren werden daher Ruthenium (III) nitrosylnitrat ($Ru(NO)(NO_3)_3$), Ruthenium (III) acetat sowie die Alkalimetallruthenate(IV) wie Natrium- und Kaliumruthenat (IV) eingesetzt.

Der Begriff "wässrig" bezeichnet hier Wasser sowie Mischungen von Wasser mit bis zu 50 Vol.-%, vorzugsweise nicht mehr als 30 vol.-% und insbesondere nicht mehr als 10 Vol.-% eines oder mehrerer mit Wasser mischbarer organischer Lösungsmittel, z.B. Mischungen von Wasser mit $C_1$-$C_4$-Alkanolen wie Methanol, Ethanol, n- oder Isopropanol. Häufig setzt man Wasser als alleiniges Lösungsmittel ein. Das wässrige Lösungsmittel wird häufig zusätzlich wenigstens eine halogenfreie Säure, z.B. Salpetersäure, Schwefelsäure, Phosphorsäure oder Essigsäure, vorzugsweise eine halogenfreie Mineralsäure, zur Stabilisierung des Rutheniumprekursors in der Lösung enthalten. In vielen Fällen setzt man daher eine mit Wasser verdünnte, halogenfreie Mineralsäure, z. B. verdünnte bis halbkonzentrierte Salpetersäure als Lösungsmittel für den Rutheniumprekursor ein. Die Konzentration des Rutheniumprekursors in den wässrigen Lösungen richtet sich naturgemäss nach der aufzubringenden Menge an Rutheniumprekursor und der Aufnahmekapazität des Trägermaterials für die wässrige Lösung und liegt in der Regel im Bereich von 0,1 bis 20 Gew.-%.

Das Trocknen kann nach den üblichen Verfahren der Feststofftrocknung unter Einhaltung der obengenannten Temperaturobergrenzen erfolgen. Die Einhaltung der erfindungsgemäßen Obergrenze der Trocknungstemperaturen ist für die Qualität, d.h. die Aktivität des Katalysators wichtig. Ein überschreiten der oben angegebenen Trocknungstemperaturen führt zu einem deutlichen Verlust an Aktivität. Ein Kalzinieren des Trägers bei höheren Temperaturen, z.B. oberhalb 300°C oder gar 400°C, wie es im Stand der Technik vorgeschlagen wird, ist nicht nur überflüssig sondern wirkt sich auch nachteilig auf die Aktivität des Katalysators aus. Zur Erreichung hinreichender Trocknungsgeschwindigkeiten erfolgt die Trocknung in der Regel bei erhöhter Temperatur, z. B. bei wenigstens 40°C, insbesondere wenigstens 70°C und speziell wenigstens 100°C.

Die Trocknung des mit dem Rutheniumprekursor getränkten Feststoffs erfolgt üblicherweise unter Normaldruck wobei zur Förderung der Trocknung auch ein verminderter Druck angewendet werden kann. Häufig wird man zur Förderung der Trocknung einen Gasstrom über bzw. durch das zu trocknende Gut leiten, z.B. Luft oder Stickstoff.

Die Trocknungsdauer hängt naturgemäss von dem gewünschten Grad der Trocknung und der Trocknungstemperatur ab und liegt in der Regel im Bereich von 2 h bis 30 h, vorzugsweise im Bereich von 4 h bis 15 h.

Vorzugsweise führt man die Trocknung des behandelten Trägermaterials soweit, dass der Gehalt an Wasser bzw. an flüchtigen Lösungsmittelbestandteilen vor der Reduktion ii) weniger als 5 Gew.-%, insbesondere nicht mehr als 2 Gew.-% und besonders bevorzugt nicht mehr als 1 Gew.-%, bezogen auf das Gesamtgewicht des Feststoffs ausmacht. Die angegebenen Gewichtsanteile beziehen sich hierbei auf den Gewichtsverlust des Feststoffs, bestimmt bei einer Temperatur von 300 °C, einem Druck von 1 bar und einer Dauer von 10 min. Auf diese Weise kann die Aktivität der erfindungsgemäßen Katalysatoren weiter gesteigert werden.

Vorzugsweise erfolgt das Trocknen unter Bewegen des mit der Prekursor-Lösung behandelten Feststoffs, beispielsweise durch Trocknen des Feststoffs in einem Drehrohrofen oder einem Drehkugelofen. Auf diese Weise kann die Aktivität der erfindungsgemäßen Katalysatoren weiter gesteigert werden.

Die überführung des nach dem Trocknen erhaltenen Feststoffs in seine katalytisch aktive Form erfolgt erfindungsgemäß durch Hydrieren des Feststoffs bei den oben angegebenen Temperaturen in an sich bekannter Weise (Schritt ii)).

Zu diesem Zweck bringt man das Trägermaterial bei den oben angegebenen Temperaturen mit Wasserstoff oder einer Mischung aus Wasserstoff und einem Inertgas in Kontakt. Der Wasserstoffpartialdruck ist für das Ergebnis der Reduktion von untergeordneter Bedeutung und wird in der Regel im Bereich von 0,2 bar bis 1,5 bar variiert werden. Häufig erfolgt die Hydrierung des Katalysatormaterials bei Wasserstoffnormaldruck im Wasserstoffstrom. Vorzugsweise erfolgt das Hydrieren unter Bewegen des in i) erhaltenen Feststoffs, beispielsweise durch Hydrieren des Feststoffs in einem Drehrohrofen oder einem Drehkugelofen. Auf diese Weise kann die Aktivität der erfindungsgemäßen Katalysatoren weiter gesteigert werden.

Im Anschluss an die Hydrierung kann der Katalysator zur Verbesserung der Handhabbarkeit in bekannter Weise passiviert werden, z.B. indem man den Katalysator kurzfristig mit einem Sauerstoffhaltigen Gas, z.B. Luft, vorzugsweise jedoch mit einer 1 bis 10 Vol.-% Sauerstoff enthaltenden Inertgasmischung behandelt.

Im erfindungsgemässen Verfahren erfolgt die Hydrierung des Monosaccharids durch Hydrieren einer wässrigen Lösung des jeweiligen Monosaccharids, bzw. im Falle des Invertzuckers als Ausgangsmaterial, der Monosaccharid-Mischung. Der Begriff "wässrig" ist hierbei in der oben definierten Weise zu verstehen. Zweckmäßigerweise wird Wasser als alleiniges Lösungsmittel verwendet, das gegebenenfalls geringe Mengen einer vorzugsweise halogenfreien Säure zur Einstellung des pH-Wertes enthält. Insbesondere setzt man das Monosaccharid als wässrige Lösung ein, die einen pH-Wert im Bereich von 4 bis 10, und speziell im Bereich von 5 bis 7 aufweist.

Die Konzentration an Monosaccharid in der flüssigen Phase kann grundsätzlich frei gewählt werden und liegt häufig im Bereich von 10 bis 80 Gew.-% und vorzugsweise im Bereich von 15 bis 50 Gew.-%, bezogen auf das Gesamtgewicht der Lösung.

Die eigentliche Hydrierung erfolgt üblicherweise in Analogie zu den bekannten Hydrierverfahren für die Herstellung von Zuckeralkoholen, wie sie im eingangs genannten Stand der Technik beschrieben werden. Hierzu wird die flüssige, das Monosaccharid enthaltende Phase mit dem Katalysator in Gegenwart von Wasserstoff in Kontakt gebracht. Der Katalysator kann dabei sowohl in der flüssigen Phase suspendiert werden (Suspensionsfahrweise) oder man führt die flüssige Phase über ein Katalysator-Fließbett (Fließbett-Fahrweise) oder ein Katalysator-Festbett (Festbettfahrweise). Die Hydrierung kann sowohl kontinuierlich als auch diskontinuierlich ausgestaltet werden. Vorzugsweise führt man das erfindungsgemäße Verfahren in Rieselreaktoren nach der Festbettfahrweise durch. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung nach der Suspensionsfahrweise als auch zur Hydrierung am Katalysator-Fließbett und am Katalysator-Festbett sind aus dem Stand der Technik bekannt, z.B. aus Ullmanns Enzyklopädie der Technischen Chemie, 4. Auflage, Band 13, S. 135 ff. sowie aus P. N. Rylander, "Hydrogenation and Dehydrogenation" in Ullmann's Encyclopedia of Industrial Chemistry, 5th ed. on CD-ROM bekannt.

In der Regel führt man die Hydrierung bei erhöhtem WasserstoffDruck, z.B. bei einem Wasserstoffpartialdruck von wenigstens 10 bar, vorzugsweise wenigstens 20 bar und insbesondere wenigstens 40 bar durch. In der Regel wird der wasserstoffpartialdruck einen Wert von 500 bar, insbesondere 350 bar nicht überschreiten. Besonders bevorzugt liegt der Wasserstoffpartialdruck im Bereich von 40 bis 200 bar. Die Reaktionstemperaturen betragen in der Regel wenigstens 40°C und werden häufig einen Wert von 250°C nicht überschreiten. Insbesondere führt man das Hydrierverfahren bei Temperaturen im Bereich von 80 bis 150°C durch.

Aufgrund der hohen Katalysatoraktivität benötigt man vergleichsweise geringe Mengen an Katalysator bezogen auf das eingesetzte Edukt. So wird man bei der diskontinuierlchen Suspensionsfahrweise in der Regel weniger als 1 mol-%, z.B. $10^{-3}$ mol-% bis 0,5 mol-% Ruthenium, bezogen auf 1 mol Zucker einsetzen. Bei kontinuierlicher Ausgestaltung des Hydrierverfahrens wird man üblicherweise das zu hydrierende Edukt in einer Menge von 0,05 bis 2 kg/(1(Katalysator) *h), insbesondere in einer Menge von 0,07 bis 0,7 kg/(1(Katalysator)*h) über den Katalysator führen.

Im erfindungsgemässen Verfahren fällt eine Lösung des Sorbits in dem jeweils eingesetzten wässrigen Lösungsmittel an, aus dem es nach bekannten Verfahren gewonnen werden kann (siehe H. Schiweck et al. "Sugar Alcohols" in Ullmann's Encyclopedia of Industrial Chemistry, 5th ed. on CD-ROM). Bei den bevorzugt erhaltenen wässrigen Reaktionsmischungen kann man das Sorbit beispielsweise durch Eindampfen mit nachfolgender Kristallisation (DE-A 2350690, EP-A 32288, EP-A 330352) oder Sprühtrocknen (DK 133603, DD 277176) gewinnen. Falls erforderlich wird zuvor der Katalysator nach üblichen Verfahren abgetrennt und die Reaktionslösung einer Entfärbung mit geeigneten Filterhilfsmitteln und/oder einer Behandlung mit Ionentauschern zur Entfernung von Metallionen, Gluconaten oder anderen organischen Säuren unterworfen.

Bei Verwendung von Invertzucker oder Fruktose wird neben Sorbit naturgemäss auch noch Mannit gebildet. Aus den dabei erhaltenen Reaktionsmischungen kann Sorbit durch selektive Kristallisation gewonnen werden.

Das erfindunggemäße Verfahren zeichnet sich zum einen durch die erreichten hohen Raum-Zeit-Ausbeuten und bei Verwendung von Glucose als Ausgangsmaterial auch durch eine hohe Produktselektivität aus. Zudem zwichnen sich die erfindungsgemäss verwendeten Rutheniumkatalysatoren durch besonders hohe Standzeiten aus, wodurch das Verfahren wirtschaftlich besonders attraktiv wird.

Selbstverständlich können die in diesem Verfahren eingesetzten Katalysatoren bei nachlassender Aktivität nach den für Edelmetallkatalysatoren wie Rutheniumkatalysatoren üblichen, dem Fachmann bekannten Methoden regeneriert werden. Hier sind z. B. die Behandlung des Katalysators mit Sauerstoff wie in der BE 882279 beschrieben, die Behandlung mit verdünnten, halogenfreien Mineralsäuren, wie in der US 4,072,628 beschrieben, oder die Behandlung mit Wasserstoffperoxid, z. B. in Form wässriger Lösungen mit einem Gehalt von 0,1 bis 35 Gew.-%, oder die Behandlung mit anderen oxidierenden Substanzen, vorzugsweise in Form halogenfreier Lösungen zu nennen. Üblicherweise wird man den Katalysator nach der Reaktivierung und vor dem erneuten Einsatz mit einem Lösungsmittel, z. B. Wasser, spülen.

Die folgenden Beispiele dienen der nähren Erläuterung der Erfindung:

I Herstellung der Katalysatoren

1. Vorschrift A: Pulverförmiger, halogenfreier Katalysator, nicht kalziniert.

Eine definierte Menge des jeweiligen Trägermaterials wurde mit der maximalen Menge einer Lösung von Ruthenium (III)nitrosylnitrat in Wasser getränkt, die vom jeweiligen Trägermaterial aufgenommen werden konnte. Die maximale vom jeweiligen Trägermaterial aufgenommene Menge war zuvor anhand einer authentischen Probe bestimmt worden. Die Konzentration der Lösung wurde jeweils so bemessen, dass die gewünschte Konzentration an Ruthenium im Trägermaterial resultierte.

Anschliessend wurde der so erhaltene Feststoff 13 h bei 120°C in einem Drehkugelofen getrocknet. Der Restwassergehalt lag unter 1 Gew.-%, bestimmt als Gewichtsverlust einer 10 min bei 300°C und 1 bar getrockneten Probe.

Der so erhaltene Feststoff wurde in einem Drehkugelofen 4 h bei 300°C im Wasserstoffstrom bei Normaldruck reduziert. Nach Abkühlen und Inertisieren mit Stickstoff wurde der Katalysator durch Überleiten von 5 Vol.-% Sauerstoff in Stickstoff über einen Zeitraum von 120 min passiviert.

2. Vorschrift B: Pulverförmiger, halogenfreier Katalysator, kalziniert.

Die Herstellung erfolgte analog Vorschrift A, jedoch wurde der nach dem Trocknen erhaltene Feststoff vor der Hydrierung 4 h auf 400°C im Luftstrom erhitzt.

3. Vorschrift C: Pulverförmiger, halogenhaltiger Katalysator, nicht kalziniert.

Die Herstellung erfolgte analog Vorschrift A, jedoch wurde anstelle von Ruthenium (III) nitrosylnitrat Ruthenium (III)chlorid eingesetzt.

4. Vorschrift D: Strangförmiger, halogenfreier Katalysator, nicht kalziniert.

Eine definierte Menge von zylindrischen Trägermaterial-Strängen (Durchmesser 4 mm, Länge 3 bis 10 mm) wurde mit der maximalen Menge einer Lösung von Ruthenium(III)nitrosylnitrat in Wasser getränkt, die vom jeweiligen Trägermaterial aufgenommen werden konnte. Die maximale vom jeweiligen Trägermaterial aufgenommene Menge war zuvor anhand einer authentischen Probe bestimmt worden. Die Konzentration der Lösung wurde jeweils so bemessen, dass die gewünschte Konzentration an Ruthenium im Trägermaterial resultierte.

Anschliessend wurden die so erhaltenen, getränkten Stränge 13 h bei 120°C in einer Drehkugelofen getrocknet. Der Restwassergehalt betrug weniger als 1 Gew.-%.

Die so erhaltenen, gerockneten Stränge wurden in einem Drehkugelofen 4 h bei 300°C im Wasserstoffstrom bei Normaldruck reduziert. Nach Abkühlen und Inertisieren mit Stickstoff wurde der so erhaltene Katalysator durch Überleiten von 5 Vol.-% Sauerstoff in Stickstoff über einen Zeitraum von 120 min passiviert.

5. Vorschrift E: strangförmiger, halogenhaltiger Katalysator, nicht kalziniert.

Die Herstellung erfolgte analog Vorschrift D, jedoch wurde anstelle von Ruthenium(III)nitrosylnitrat Ruthenium(III)chlorid eingesetzt.

Tabelle 1: Katalysatoren

| Katalysator Nr. | Rutheniumgehalt [Gew.-%] | Vorschrift | Träger |
|---|---|---|---|
| K1 | 1 | A | $SiO_2$ Pulver[1] |
| K2 (V) | 1 | C | $SiO_2$ Pulver[1] |
| K3 (V) | 1 | B | $SiO_2$ Pulver[1] |
| K4 | 1 | A | $SiO_2$ Pulver[2] |
| K5 | 1 | A | $SiO_2$ Pulver[3] |
| K6 | 1 | A | $SiO_2$ Pulver[4] |
| K7 (V) | 1 | A | $TiO_2$ Pulver[5] |
| K8 (V) | 1 | A | $ZrO_2$ Pulver[6] |
| K9 (V) | 1 | A | $\gamma$-$Al_2O_3$ Pulver[7] |
| K10 (V) | 1 | A | Aktivkohle[8] |
| K11 (V) | 1 | A | H-ZSM 5 [9] |
| K12 (V) | 1 | A | Magnesiumoxid[10] |
| K13 (V) | 1 | A | Alumosilikat[11] |
| K14 (V) | 1 | A | $\theta$-$Al_2O_3$ Pulver[12] |
| K15 | 1 | D | $SiO_2$ Stränge[13] |
| K16 (V) | 1 | E | $SiO_2$ Stränge[13] |

V Vergleichskatalysator

1) Kieselgel-Pulver mit einem $SiO_2$-Gehalt > 99,95 Gew.-%,
einer spezifischen BET-Oberfläche von 523 m$^2$/g,
einer Wasseraufnahme von 1,4 ml/g,
einem Porenvolumen von 0,75 ml/g (ermittelt durch Stickstoffporometrie nach DIN 66134),
einer definierten Porengröße von 60 Å
einer Teilchengrösse von 63 bis 200 $\mu$m;

2) Kieselgel-Pulver mit einem $SiO_2$-Gehalt > 99,95 Gew.-%,
einer spezifischen BET-Oberfläche von 317 m$^2$/g,
einer Wasseraufnahme von 1,4 ml/g,
einer Teilchengrösse < 63 $\mu$m;

3) Kieselgel-Pulver mit einem $SiO_2$-Gehalt > 99,95 Gew.-%,
einer spezifischen BET-Oberfläche von 270 m$^2$/g,
einer Wasseraufnahme von 1,5 ml/g,
einer Teilchengrösse < 63 $\mu$m;

4) Kieselgel-Pulver mit einem $SiO_2$-Gehalt > 99,5 Gew.-%,
einer spezifischen BET-Oberfläche von 68 m$^2$/g,
einer Wasseraufnahme von 1,04 ml/g,
einer Teilchengrösse < 63 $\mu$m;

5) Titandioxid-Pulver mit einem $TiO_2$-Gehalt > 99,9 Gew.-%,
einer spezifischen BET-Oberfläche von 325 m$^2$/g,
einer Wasseraufnahme von 0,84 ml/g,

einer Teilchengrösse < 63 $\mu$m;

6) Zirkondioxid-Pulver mit einem $ZrO_2$-Gehalt > 99,5 Gew.-%,
einer spezifischen BET-Oberfläche von 138 $m^2$/g,
einer Wasseraufnahme von 0,7 ml/g,
einer Teilchengrösse von < 63 $\mu$m;

7) gamma-Aluminiumoxid-Pulver mit einem $Al_2O_3$-Gehalt > 99,5 Gew.-%,
einer spezifischen BET-Oberfläche von 226 $m^2$/g,
einer Wasseraufnahme von 1,1 ml/g,
einem Porenvolumen von 0,54 ml/g,
einer Teilchengrösse < 63 $\mu$m;

8) Aktivkohle Norit CA1 mit
einer spezifischen BET-Oberfläche von 1306 $m^2$/g,
einer Wasseraufnahme von 1,7 ml/g;

9) H-ZSM 5 Zeolith, Typ ZSM 5 der Fa. Vetikon.

10) Magnesiumoxid mit einem MgO-Gehalt > 99 Gew.-%,
einer spezifischen BET-Oberfläche von 81 $m^2$/g,
einer Wasseraufnahme von 3,2 ml/g,
einer Teilchengrösse < 63 $\mu$m;

11) Alumosilikat, mit einem $Al_2O_3$/$SiO_2$-Verhältnis von 30/70
einer spezifischen BET-Oberfläche von 482 $m^2$/g,
einem Porenvolumen von 0,33 ml/g,
Wasseraufnahme von 0,57 ml/g,
einer Teilchengrösse von < 63 $\mu$m;

12) theta-Aluminiumoxid-Pulver mit einem $Al_2O_3$-Gehalt > 99,95 Gew.-%,
einer spezifischen BET-Oberfläche von 80 $m^2$/g,
einer Wasseraufnahme von 1,05 ml/g,
einem Porenvolumen von 0,67 ml/g (DIN 66134),
einer Teilchengrösse < 100 $\mu$m;

13) Kieselgel-Stränge (d 4 mm, 1 l bis 10 mm) aus Kieselgel mit
einem $SiO_2$-Gehalt > 99,5 Gew.-% (0,3 Gew.-% $Na_2O$),
einer spezifischen BET-Oberfläche von 169 $m^2$/g,
einer Wasseraufnahme von 0,95 ml/g,
einem Porenvolumen von 0,7 ml/g (DIN 66134).

II. Hydrierung von D-Glucose in Suspensionsfahrweise (Beispiel 1, Vergleichsbeispiele V1 bis V3)

Allgemeine Hydriervorschrift.

In einem 2,5 l Autoklaven mit Rührer, Vorrichtungen zur Probenentnahme und einer Druckhaltung für Wasserstoff wurden 1200 ml einer 30 Gew.-% Lösung von D(+) Glucose in Wasser zusammen mit 3 g des jeweiligen Katalysators vorgelegt. Der Katalysator wurde mit Stickstoff inertisiert. Anschließend presste man 50 bar Wasserstoff auf und erwärmte den Autoklaven auf 120°C. Während der Reaktion wurde mit 1000 U/min gerührt. Zur Ermittlung des Umsatzes wurden während der Reaktion alle 20 min Proben entnommen und mittels HPLC der Gehalt an Sorbit und Mannit bestimmt. Die Umsetzung wurde spätestens nach 20 h abgebrochen. In Tabelle 2 ist die Zeitdauer angegeben, die zur Erreichung einer maximalen Ausbeute erforderlich ist. Ausserdem ist die Selektivität hinsichtlich der Bildung von Sorbit sowie die Bildung von Mannit als Nebenprodukt angegeben.

EP 1 412 083 B1

Tabelle 2:

| Beispiel | Kat. Nr. | Träger | t-max. [h] | Umsatz [%] | Selektivität [%] | Mannit [%] |
|---|---|---|---|---|---|---|
| 1 | K1 | $SiO_2$ | 1,5 | 99,7 | 97,9 | 1,08 |
| V1 | K2(V) | $SiO_2$ | 3 | 99,8 | 95,6 | 1,34 |
| V2 | K3(V) | $SiO_2$ | 10 | 99,4 | 99,1 | n.b. |
| V3 | K14(V) | $\theta\text{-Al}_2O_3$ | 20 | 98 | 98,7 | 0,51 |

III Hydrierung von D-Glucose in Suspensionsfahrweise (Beispiele 2 bis 5, Vergleichsbeispiele V4 bis V10)

Analog der unter II angegebenen allgemeinen Hydriervorschrift wurden wurden 180 ml einer 30 Gew.-% Lösung von D-Glucose in Wasser zusammen mit 0,9 g des jeweiligen Katalysators in einem 300 ml-Autoklaven bei 100 bar Wasserstoff und einer Temperatur von 90°C hydriert. Umsatz und Selekivität wurden wie unter II beschrieben mittels HPLC ermittelt. In Tabelle 3 ist die Zeitdauer angegeben, die zur Erreichung einer maximalen Ausbeute erforderlich ist. Ausserdem ist die Selektivität hinsichtlich der Bildung von Sorbit angegeben.

Tabelle 3:

| Beispiel | Kat. Nr. | Träger | t-max. [h] | Umsatz [%] | Selektivität [%] |
|---|---|---|---|---|---|
| V4 | K7(V) | $TiO_2$ | 22 | 100 | 95,5 |
| V5 | K8(V) | $ZrO_2$ | 44 | 68 | 92,9 |
| V6 | K9(V) | $\gamma\text{-Al}_2O_3$ | 17 | 99,6 | 97,8 |
| V7 | K10(V) | A-Kohle | 20 | 100 | >99,5 |
| V8 | K11(V) | H_ZSM 5 | 19 | 100 | 97,0 |
| V9 | K12(V) | MgO | 18 | 92 | 40 |
| V10 | K13(V) | Alumosilikat | 19 | 100 | 95,7 |
| 2 | K4 | $SiO_2$ | 2 | 100 | >99,5 |
| 3 | K5 | $SiO_2$ | 4 | 100 | >99,5 |
| 4 | K1 | $SiO_2$ | 6 | 100 | >99,5 |
| 5 | K6 | $SiO_2$ | 5 | 100 | >99,5 |

III Hydrierung von D-Glucose am Katalysatorfestbett (Beispiel 6 und Vergleichsbeispiel V11)

Als Reaktor diente ein beheizbares Reaktionsrohr aus Edelstahl das mit Katalysator gefüllt war. Die Reaktionsanordnung wies eine Zulaufpumpe für die Edukte, einer Kreislaufpumpe, Vorrichtungen für die Probenentnahme sowie einem Abscheider mit Standregelung und einer Abgasregelung.

In dieser Reaktionsanordnung wurden 240 ml einer 30 gew.-%igen Lösung des jeweiligen Mono- bzw. Disaccharids bei einer Temperatur von 100°C und einem Wasserstoffdruck von 50 bar mit einer Geschwindigkeit von 50 ml/(g(Katalysator)*h) im Kreis gefahren und währenddessen mittels der unter II beschriebenen Analytik die Abnahme des Edukts, die Zunahme des Produkte und die Bildung von Nebenprodukten bestimmt. Bei erreichen eines Umsatzes von 99,4 % wurde die Umsetzung abgebrochen. Die zur Erreichung der maximalen Ausbeute erforderliche Kontaktzeit ist in Tabelle 4 zusammen mit der Selektivität angegeben.

**Kontaktzeit = Vol.(Lösung)/Vol.(Reaktionsrohr)*Reaktionszeit**

9

Tabelle 4:

| Beispiel | Kat. Nr. | Träger | t-Kontakt [h] | Umsatz [%] | Selektivität [%] | Mannit [%] |
|---|---|---|---|---|---|---|
| 6 | K15 | $SiO_2$ | 1,2 | 99,5 | 97,4 | 0,7 |
| V11 | K16(V) | $SiO_2$ | 1,2 | 99,5 | 96,8 | 2,1 |

**Patentansprüche**

1. Verfahren zur Herstellung von Sorbit durch katalytische Hydrierung einer wässrigen Lösung eines Monosaccharids, das bei der Hydrierung Sorbit bildet, an einem Ruthenium-Katalysator, enthaltend Ruthenium auf einem Trägermaterial auf Basis von amorphem Siliziumdioxid, wobei der Gehalt an Ruthenium 0,2 bis 10 Gew.-%, bezogen auf das Trägermaterial, beträgt und das Trägermaterial zu wenigstens 90 Gew.-%, bezogen auf das Trägermaterial, aus Siliziumdioxid besteht und weniger als 10 Gew.-%, kristalline Siliziumdioxid-Phasen aufweist, wobei der Katalysator erhältlich ist durch;

   i) ein oder mehrfaches Behandeln des Trägermaterials auf Basis von amorphem Siliziumdioxid mit einer wässrigen Lösung einer Rutheniumverbindung, ausgewählt unter Ruthenium((III)nitrosylnitrat, Ruthenium((III)acetat und Alkalimetallruthenaten(IV), wobei die wässrige Lösung, bezogen auf ihr Gesamtgewicht, weniger als 500 ppm Halogen enthält, und anschliessendes Trocknen des behandelten Trägermaterials bei einer Temperatur unterhalb 200°C,
   ii) Reduktion des in i) erhaltenen Feststoffs mit Wasserstoff bei einer Temperatur im Bereich von 100 bis 350°C,

   wobei man Schritt ii) unmittelbar im Anschluss an schritt i) durchführt.

2. Verfahren Anspruch 1, worin das Trägermaterial eine BET-Oberfläche im Bereich von 50 bis 700 m$^2$/g aufweist.

3. Verfahren nach Anspruch 1, **dadurch gekennzeichnet, dass** der Ruthenium-Katalysator weniger als 0,05 Gew.-% Halogen, bezogen auf das Gesamtgewicht des Katalysators, enthält, und worin das Ruthenium als elementares Ruthenium vorliegt, das auf dem Trägermaterial in atomar-disperser Form und/oder in Form von Ruthenium-Partikeln vorliegt, wobei der Katalysator im Wesentlichen keine Ruthenium-Partikel und/oder Agglomerate mit Durchmessern oberhalb 10 nm aufweist.

4. Verfahren nach einem der vorhergehenden Ansprüche, **dadurch gekennzeichnet, dass** man das Monosaccharid als wässrige Lösung einsetzt, die einen pH-Wert im Bereich von 4 bis 10 aufweist.

5. Verfahren nach einem der vorhergehenden Ansprüche, **dadurch gekennzeichnet, dass** das Monosaccharid Glucose ist.

6. Verfahren nach einem der vorhergehenden Ansprüche, **dadurch gekennzeichnet, dass** man die Hydrierung bei einem Wasserstoffpartialdruck im Bereich von 10 bis 500 bar durchführt.

7. Verfahren nach einem der vorhergehenden Ansprüche, **dadurch gekennzeichnet, dass** man die Hydrierung bei einer Temperatur im Bereich von 40 bis 250°C durchführt.

8. Verfahren nach einem der vorhergehenden Ansprüche, **dadurch gekennzeichnet, dass** man die Hydrierung an einem Katalysatorfestbett durchführt.

9. verfahren nach einem der Ansprüche 1-7, **dadurch gekennzeichnet, dass** man die Hydrierung in flüssiger Phase, enthaltend den Katalysator in Form einer Suspension, durchführt.

**Claims**

1. A process for preparing sorbitol by catalytic hydrogenation of an aqueous solution of a monosaccharide, which forms sorbitol on hydrogenation, over a ruthenium catalyst comprising ruthenium on a support material based on amorphous silicon dioxide, wherein the ruthenium content is from 0.2 to 10% by weight, based on the support material, and the support material comprises at least 90% by weight, based on the support material, of silicon dioxide and has less than 10% by weight of crystalline silicon dioxide phases, and the catalyst is obtainable by:

> i) treating the support material based on amorphous silicon dioxide with an aqueous solution of a ruthenium compound selected from among ruthenium(III) nitrosyl nitrate, ruthenium(III) acetate and potassium ruthenate (IV), with the aqueous solution, based on its total weight, comprising less than 500 ppm of halogen, and subsequently drying the treated support material at below 200°C,
> ii) reducing the solid obtained in i) by means of hydrogen at from 100 to 350°C,

where step ii) is carried out directly after step i).

2. The process claim 1, wherein the support material has a BET surface area in the range from 50 to 700 m$^2$/g.

3. The process as claimed in claim 1, wherein the ruthenium catalyst comprises less than 0.05% by weight of halogen, based on the total weight of the catalyst, and the ruthenium is present as elemental ruthenium which is present in atomically disperse form and/or in the form of ruthenium particles on the support material, where the catalyst comprises essentially no ruthenium particles and/or agglomerates having diameters above 10 nm.

4. The process as claimed in any of the preceding claims, wherein the monosaccharide or oligosaccharide is used as an aqueous solution having a pH in the range from 4 to 10.

5. The process as claimed in any of the preceding claims, wherein the monosaccharide is glucose.

6. The process as claimed in any of the preceding claims, wherein the hydrogenation is carried out at a hydrogen partial pressure in the range from 10 to 500 bar.

7. The process as claimed in any of the preceding claims, wherein the hydrogenation is carried out at from 40 to 250°C.

8. The process as claimed in any of the preceding claims, wherein the hydrogenation is carried out over a fixed catalyst bed.

9. The process as claimed in any of claims 1 to 7, wherein the hydrogenation is carried out in a liquid phase in which the catalyst is present in the form of a suspension.

**Revendications**

1. Procédé de fabrication de sorbitol par hydrogénation catalytique d'une solution aqueuse d'un monosaccharide, qui forme pendant l'hydrogenation du sorbitol, sur un catalyseur à base de ruthénium contenant du ruthénium sur un matériau support à base de dioxyde de silicium amorphe, le contenu du ruthénium étant de 0,2 à 10 % en poids par rapport au matériau support, et ledit matériau support étant constitué d'au moins 90 % en poids, par rapport au matériau support, de dioxyde de silicium et comprenant moins de 10 % en poids de phases cristallines de dioxyde de silicium, ledit catalyseur étant obtenu par :

> i) un ou plusieurs traitements du matériau support à base de dioxyde de silicium amorphe avec une solution aqueuse d'un composé de ruthénium, choisi parmi le nitrate de nitrosyle de ruthénium (III), l'acétate de ruthénium (III) et le ruthénate (IV) de potassium, ladite solution aqueuse contenant, par rapport à son poids total, moins de 500 ppm d'halogène, puis séchage ultérieur du matériau support traité à une température inférieure à 200 °C,
> ii) réduction du solide obtenu en i) avec de l'hydrogène à une température de 100 à 350 °C,

l'étape ii) étant réalisée immédiatement après l'étape i).

2. Procédé selon la revendication 1, **caractérisée en ce que** le matériau support présente une surface BET de 50 à 700 m$^2$/g.

3. Procédé selon la revendication 1, **caractérisée en ce que** le catalyseur à base de ruthénium contient moins de 0,05 % en poids d'halogène par rapport au poids total du catalyseur et **en ce que** le ruthénium se présente comme ruthénium élémentaire, qui est présent sur le matériau support sous forme atomique dispersée et/ou sous forme de particules de ruthénium, ledit catalyseur ne comportant essentiellement pas de particules et/ou d'agglomérats de ruthénium ayant un diamètre supérieur à 10 nm.

4.  Procédé selon l'une quelconque des revendications précédentes, **caractérisé en ce que** le mono- ou l'oligosaccharide est utilisé sous forme d'une solution aqueuse dont le pH est de 4 à 10.

5.  Procédé selon l'une quelconque des revendications précédentes, **caractérisé en ce que** le monosaccharide est le glucose.

6.  Procédé selon l'une quelconque des revendications précédentes, **caractérisé en ce que** l'hydrogénation est réalisée à une pression partielle en hydrogène de 10 à 500 bars.

7.  Procédé selon l'une quelconque des revendications précédentes, **caractérisé en ce que** l'hydrogénation est réalisée à une température de 40 à 250 °C.

8.  Procédé selon l'une quelconque des revendications précédentes, **caractérisé en ce que** l'hydrogénation est réalisée sur un lit catalytique solide.

9.  Procédé selon l'une quelconque des revendications 1 à 7, **caractérisé en ce que** l'hydrogénation est réalisée en phase liquide contenant le catalyseur sous forme d'une suspension.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4380680 A
- US 4487980 A
- US 4413152 A
- US 4471144 A
- US 4503274 A
- US 3963788 A
- US 3963789 A
- FR 2526782 A
- WO 02100537 A
- WO 02100536 A
- WO 02100538 A
- DE 10128205
- DE 2350690 A
- EP 32288 A
- EP 330352 A
- DK 133603
- DD 277176
- BE 882279
- US 4072628 A

**In der Beschreibung aufgeführte Nicht-Patentliteratur**

- *Ullmanns Enzyklopädie der Technischen Chemie,* vol. 13, 135 ff